# EUROPEAN PATENT SPECIFICATION

(11) **EP 1 721 009 B1**
(45) Date of publication and mention of the grant of the patent: **22.10.2008**
(21) Application number: 05720073.5
(22) Date of filing: 25.02.2005
(51) Int. Cl.: C12Q 1/68, B01L 3/00

(54) **HYBRIDIZATION METHOD AS WELL AS HYBRIDIZATION MICROARRAY AND HYBRIDIZATION KIT**
HYBRIDISIERUNGSVERFAHREN SOWIE HYBRIDISIERUNGS-MIKROARRAY UND HYBRIDISIERUNGSKIT
PROCEDE D'HYBRIDATION AINSI QUE PUCE ADN D'HYBRIDATION ET KIT D'HYBRIDATION

(30) Priority: 01.03.2004 JP 2004056511
(43) Date of publication of application: 15.11.2006
(73) Proprietor: Kurashiki Boseki Kabushiki Kaisha, Kurashiki-shi Okayama 710-0054 (JP)
(72) Inventor: MIYAGAWA, Isao, c/o KURASHIKI BOSEKI KK, Neyagawa-shi, Osaka, 5720823 (JP); ISAGAWA, Yoshihiko, c/o KURASHIKI BOSEKI KK, Neyagawa-shi, Osaka, 5720823 (JP); MORISHITA, Atsushi, c/o KURASHIKI BOSEKI KK, Neyagawa-shi, Osaka, 5720823 (JP)
(74) Representative: Setna, Rohan P.
(86) International application number: PCT/JP2005/003801
(87) International publication number: WO 2005/083119

(56) References cited:
- WO-A-02/072264
- WO-A-03/083127
- US-A1- 2002 127 589
- US-A1- 2002 187 476
- US-A1- 2003 194 709

### SEQUENCE LISTING

<110> KURASHIKI BOSEKI KABUSHIKI KAISHA
<120> Hybridization method as well as Hybridization Microarray an d Hybridization Kit
<130> 904465
<150> JP2004-056511
   <151> 2004-03-01
<160> 4
<170> PatentIn version 3.1
<210> 1
   <211> 70
   <212> DNA
   <213> Artificial
<220>
   <223> 70mer probe of glyceraldehyde-3-phosphate dehydrogenase
<400> 1
   gctgagaacg ggaagcttgt catcaatgga aatcccatca ccatcttcca ggagcgagat 60
   ccctccaaaa 70
<210> 2
   <211> 70
   <212> DNA
   <213> Artificial
<220>
   <223> 70 mer probe of endonuclease G
<400> 2
   tggagcgctt cctggtgccc atcgagagca ttgagcgggc ttcggggctg ctctttgtgc 60
   caaacatcct 70
<210> 3
   <211> 70
   <212> DNA
   <213> Artificial
<220>
   <223> 70 mer probe of pU18
<400> 3
   cgactctaga ggatccccgg gtaccgagct cgaattcgta atcatggtca tagctgtttc
   60
   ctgtgtgaaa
   70
<210> 4
   <211> 70
   <212> DNA
   <213> Artificial
<220>
   <223> 70mer probe of Lamda phage DNA
<400> 4
   gtccttctcg gtgcatgcca ctgttgccaa tgacctgcct aggaattggt tagcaagtta
   60
   ctaccggatt
   70

## Description

### Technical Field

The present invention relates to a hybridization method for simultaneously hybridizing a plurality of sample biopolymers with probe biopolymers as well as a hybridization microarray and a hybridization kit.

### Background Art

In general, a method of hybridizing a probe of nucleic acid or protein having a known sequence with sample DNA (generally a sample biopolymer) labeled with a fluorescent material is frequently employed in order to identify/fractionate a molecule in vivo, particularly for detecting target DNA or presence/absence of gene DNA. More specifically, this method is carried out with a DNA chip (generally a microarray) formed by immobilized probe DNA (generally a probe biopolymer)on a slide glass.

In order to efficiently hybridize the sample DNA with the probe DNA in a small quantity of sample DNA solution, two types of methods are generally employed as follows: In the first method, a solution containing sample DNA labeled with a fluorescent material is dripped on a slide glass to which probe DNA is immobilized, and thereafter covered with a glass coverslip. This is stored in a closed vessel referred to as a hybridization chamber along with a humectant and thereafter kept at a temperature of about 65°C for 8 to 16 hours, to be hybridized. When the sample DNA is bound to the probe DNA, the sample DNA is also immobilized along with the probe DNA, whereby the hybridized sample DNA can be detected by exciting the fluorescent material labeled on the immobilized sample DNA with excitation light from a light source and detecting emitted fluorescence.

In the second method, hybridization is performed by previously forming a separable vessel capable of receiving a solution on a slide glass to which probe DNA is immobilized, injecting a solution containing sample DNA labeled with a fluorescent material, closing the vessel by sealing an injection port and thereafter keeping the temperature at about 65°C for 8 to 16 hours. When the sample DNA is bound to the probe DNA, the sample DNA is also immobilized along with the probe DNA. The hybridized DNA can be detected by pealing the vessel off, washing the slide glass, thereafter exciting the fluorescent material labeled on the immobilized sample DNA with excitation light from a light source and detecting emitted fluorescence.

In the first method of the prior art, experience and technique are required for placing the glass coverslip on the solution containing the sample DNA dripped on the slide glass so that no bubbles are introduced into the clearance between the glass coverslip and the slide glass. If bubbles are introduced into the clearance, hybridization may not be correctly carried out.

Further, the glass coverslip placed on the slide glass is so closely in contact with the slide glass through the hybridization solution that the spot of the probe DNA may be damaged and no normal spot can be detected if the glass coverslip is moved. However, the glass coverslip is extremely thin and lightweight, and the operation of placing the same on a home position of the solution containing the sample DNA dripped on the slide glass requires experience and technique.

In addition, the glass coverslip must be correctly placed on the position of the slide glass to which the probe DNA is immobilized, and hence no small glass coverslip having a possibility of hindering the operation can be used. More specifically, the limit is two glass coverslips on a single slide glass. This means that only two samples can be hybridized on a single slide glass. Further, the volume of the hybridization solution may be varied with the composition of the humectant, leading to contamination of the two samples on the slide glass.

In the second method of the prior art, the separable vessel cannot be completely filled with the solution but air frequently remains in the vessel, to result in incorrect hybridization.

An object of the present invention is to provide a method of simply and reliably hybridizing a sample biopolymer and probe DNA with each other without using a glass coverslip and a closed vessel in consideration of the aforementioned problems.

Another object of the present invention is to provide a method of simply and reliably hybridizing a plurality of sample biopolymers and probe DNA with each other in consideration of the aforementioned problems.

### Disclosure of the Invention

The present invention, which has been proposed in order to solve the aforementioned problems, is a hybridization method carried out in a closed vessel storing a solution having the same vapor pressure as a solution containing a sample for hybridizing the sample biopolymer and a probe biopolymer with each other in a state that the solution containing the sample biopolymer is in contact with only a slide glass to which the probe biopolymer is immobilized. As the quantity of the solution having the same vapor pressure as the solution containing the said sample biopolymer, the larger one of a quantity obtained by adding at least 100 µL to the quantity of water vapor saturating the volume of the closed vessel and at least five times the quantity of the solution containing the sample biopolymer is selected.

The hybridization method according to the present invention is preferably carried out on a slide glass constituted of a hydrophilic region having a surface to which a plurality of probe biopolymers are immobilized and a hydrophobic region to which no probe biopolymer is immobilized around the hydrophilic region.

Further, the slide glass employed in the present invention is preferably a microarray formed by arranging a plurality of hydrophilic regions to which a plurality of probe biopolymers are immobilized with a hydrophobic region to which no probe biopolymer is immobilized formed around the arranged plurality of hydrophilic regions.

The present invention also provides a hybridization microarray to be applied to the aforementioned hybridization method according to the present invention, which is formed by arranging a plurality of hydrophilic regions to which a plurality of probe biopolymers are immobilized with a hydrophobic region to which no probe biopolymer is immobilized formed around the arranged plurality of hydrophilic regions.

The present invention further provides a hybridization kit to be applied to the aforementioned hybridization method according to the present invention, comprising a microarray formed by arranging a plurality of hydrophilic regions to which a plurality of probe biopolymers are immobilized with a hydrophobic region to which no probe biopolymer is immobilized formed around the arranged plurality of hydrophilic regions and a closed vessel having an internal space capable of storing the said microarray.

### Brief Description of the Drawings

Fig. 1 schematically shows an exemplary microarray 1 preferably employed for a hybridization method according to the present invention.
Fig. 2 schematically shows a state of dripping solutions 5 containing sample biopolymers on hydrophilic regions 2 of the microarray 1 shown in Fig. 1.
Fig. 3 schematically shows an exemplary closed vessel for carrying out the hybridization method according to the present invention.

### Best Modes for Carrying Out the Invention

One of the features of the hybridization method according to the present invention resides in that a sample biopolymer and a probe biopolymer are hybridized with each other in a state that a solution containing the sample biopolymer is in contract with only a slide glass to which the probe biopolymer is immobilized. The wording "in contact with only a slide glass" indicates such a state that the solution containing the sample biopolymer is not in contact with a glass coverslip or the like generally required for hybridization. According to the present invention, hybridization can be performed without requiring any structure such as a glass coverslip required in the prior art for efficiently hybridizing a probe biopolymer with a sample biopolymer, so that hybridization can be extremely simply and reliably carried out.

Throughout this specification, the term "sample biopolymer" indicates a biopolymer that can be identified/fractionated by hybridization, including DNA, RNA, peptide or protein, for example.

A proper solvent generally widely employed in this field can be selected as a solvent in the solution containing the sample biopolymer in response to the type of the sample biopolymer. An aqueous solution containing a detergent and salt is employed when the sample biopolymer is DNA, for example, and it is preferable to use SDS as the detergent and SSC as the aqueous solution containing salt in particular, in consideration of flexibility of a reagent and stability of hybridization reaction. For example, 5 × SSC containing 0.5 % of SDS can be listed. The sample biopolymer is preferably so prepared that the concentration in this solution, not particularly limited if the concentration is sufficient for detection, is in the range of 1 ng/µL to 1 µg/µL since the sample biopolymer is generally valuable.

The sample biopolymer is generally labeled for detection after hybridization. As to the label, a proper well-known label such as a fluorescent label, an organic dye label, a chemiluminescent label, a radioactive label or an antibody label can be listed, and the fluorescent label is particularly preferable in view of safety and simplicity in detection.

Throughout this specification, the term "probe biopolymer" indicates a biopolymer hybridizable with the sample biopolymer and immobilizable onto the slide glass, and includes DNA, RNA, protein or peptide, for example.

As to immobilization of the probe biopolymer onto the slide glass in the present invention, an agent for immobilizing the biopolymer generally widely employed in this field can be properly selected in response to the type of the probe biopolymer. Poly-L-lysine or aminosilane can be listed as this agent.

The slide glass employed in the hybridization method according to the present invention is not limited to that of glass but may be made of plastic or metal, and more preferably made of a material having no biochemical activity. The slide glass is preferably made of glass since a hydrophilic region and a hydrophobic region described later can be easily formed on the surface thereof.

Another feature of the hybridization method according to the present invention resides in that the aforementioned hybridization is carried out in a closed vessel storing a solution having the same vapor pressure as the solution containing the sample biopolymer (this solution is hereinafter referred to as "humectant"). In other words, hybridization is carried out by dripping the solution containing the sample biopolymer onto the slide glass and introducing this slide glass in the closed vessel (hybridization chamber) storing the humectant having the same vapor pressure as the solution containing the sample biopolymer. A microarray is stored in the closed vessel so that the solution containing the sample biopolymer is not in contact with the humectant. As the quantity of the humectant, the larger one of a quantity obtained by adding at least 100 µL to the quantity of water vapor saturating the volume of the closed vessel and at least five times the quantity of the solution containing the sample biopolymer is selected. Vapor pressure depression of a solvent caused by a nonvolatile solute is proportionate to the molar concentration of the solute, and hence the wording "the same vapor pressure" indicates "the same solute molar concentration" in the present invention. The wording "the same solute molar concentration" indicates that the difference between the molar concentration of the solute contained in the solution containing the sample biopolymer and the molar concentration of the solute contained in the humectant is -10 to +8 % (more preferably, -5 to +5 %).

In order to employ the humectant having the same vapor pressure as the solution containing the sample biopolymer as described above, a solution of the same composition as the said solution except that the sample biopolymer is contained may be employed as the humectant. While a solvent containing 5 × SSC containing 0.5 % of SDS is preferably employed as the solution containing the sample biopolymer as hereinabove described if the sample biopolymer is DNA, these vapor pressures can be rendered identical to each other by preparing the humectant to also contain 5 × SSC containing 0.5 % of SDS. In the solution containing the sample biopolymer, the concentration of the sample biopolymer, which is extremely low as compared with the salt concentration in this solution, is included in the aforementioned difference in the same range and ignorable.

Hybridization can be completed without drying the solution containing the sample biopolymer on the slide glass or without overflowing the solution by increasing the volume thereof by carrying out the hybridization in the closed vessel storing the humectant having the same vapor pressure as the solution containing the sample biopolymer as described above. If the vapor pressure of the humectant is extremely lower than the vapor pressure of the solution containing the sample biopolymer in the hybridization method according to the present invention, remarkable reduction of the quantity of the solution containing the sample biopolymer or exsiccation of this solution results from vapor diffusion during hybridization, such that the hybridization cannot be normally carried out. If the vapor pressure of the humectant is extremely higher than the vapor pressure of the solution containing the sample biopolymer, on the other hand, the quantity of the solution containing the sample biopolymer is so remarkably increased that this solution overflows and the hybridization cannot be normally carried out.

In the hybridization method according to the present invention, the said slide glass is preferably constituted of a hydrophilic region having a surface to which a plurality of probe biopolymers are immobilized and a hydrophobic region, to which no probe biopolymer is immobilized, around the hydrophilic region. Thus, the solution containing the sample biopolymer dripped on the hydrophilic region to which the probe biopolymers are immobilized on the slide glass is held on the hydrophilic region without flowing out toward the hydrophobic region. The term "hydrophilic" indicates that a droplet contact angle with respect to water (angle between the tangent of a droplet surface on the boundary between the contact surfaces of a droplet placed on a planar object surface and the object surface and the said object surface) measured through an image processing type contact angle meter (contact angle meter CA-X by Kyowa Interface Science Co., Ltd.) is less than 90°, and the term "hydrophobic" indicates that the said droplet contact angle is at least 90°.

As to a method of forming the aforementioned hydrophilic region and the hydrophobic region on the slide glass, the hydrophobic region is so formed that the hydrophilic region is formed on a desired region if the slide glass is made of glass, for example, since the surface is originally hydrophilic. The hydrophobic region is formed by a method of printing water-repellent fluororesin ink, for example. A slide glass printed with such water-repellent fluororesin ink is commercially available (trade name: water-repellent printing slide glass by Matsunami Glass Ind. Ltd.). The method of forming the hydrophobic region is not restricted to the aforementioned method employing water-repellent fluororesin ink, but the hydrophobic region may alternatively be formed by applying and hardening silicone resin, for example.

As to the slide glass employed in the present invention, an agent for immobilizing the biopolymers is preferably formed on the surface of the hydrophilic region, and this agent is preferably not formed on the hydrophobic region around the same. Thus, the probe biopolymer can be immobilized to only the hydrophilic region. The aforementioned poly-L-lysine or aminosilane can be illustrated as the agent.

While the slide glass employed in the present invention may have at least each of the aforementioned hydrophilic and hydrophobic regions, a microarray formed by arranging a plurality of hydrophilic regions with a hydrophobic region formed to enclose these hydrophilic regions is preferably employed. Hybridization can be simultaneously carried out on a plurality of types of sample biopolymers on the same slide glass by carrying out the hybridization method according to the present invention with the microarray formed by arranging a plurality of hydrophilic regions to which probe biopolymers are immobilized.

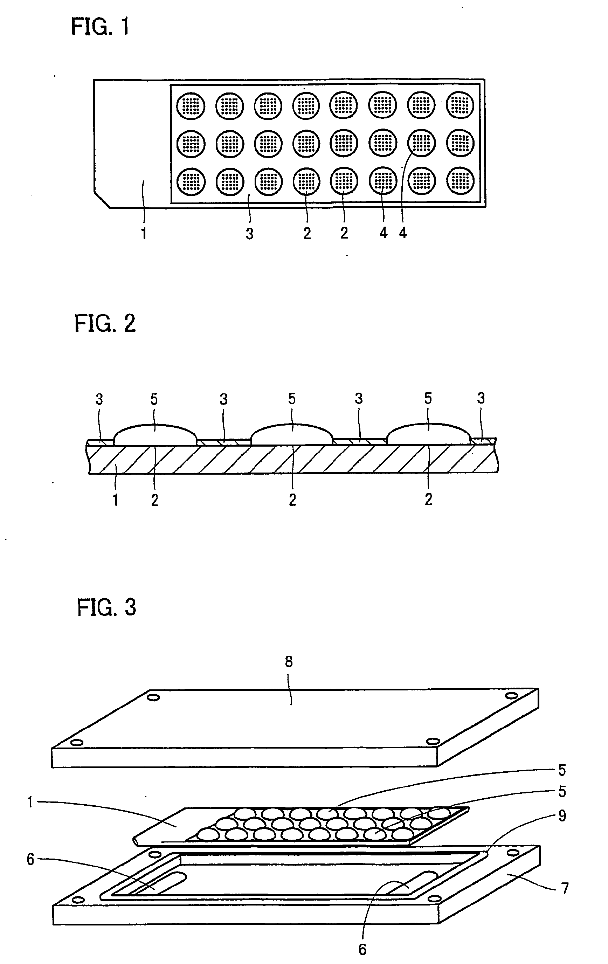

Fig. 1 schematically illustrates an exemplary microarray 1 preferably employed for the hybridization method according to the present invention. The microarray 1 shown in Fig. 1 has 24 hydrophilic regions 2 and a hydrophobic region 3 formed to enclose these hydrophilic regions 2 on the surface thereof. A plurality of probe biopolymers are immobilized to each hydrophilic region 2 (24 probe biopolymers are spotted on each hydrophilic region 2 in the example shown in Fig. 1). According to the present invention, sample biopolymers and probe biopolymers are preferably hybridized with each other with the microarray selectively formed with the plurality of hydrophilic regions 2 to which probe biopolymers are immobilized and the hydrophobic region 3 enclosing the hydrophilic regions 2 on the surface of a slide glass.

Fig. 2 schematically shows a state of dripping solutions 5 containing sample biopolymers on the hydrophilic regions 2 of the microarray 1 shown in Fig. 1. When solutions containing sample biopolymers are dripped on arbitrary hydrophilic regions of the microarray 1, these solutions spreading on the hydrophilic regions are inhibited by the hydrophobic region from further spreading over the hydrophilic regions, so that the solutions containing the sample biopolymers dripped onto adjacent hydrophilic regions are not mixed with each other. When the solutions containing the sample biopolymers are dripped on the hydrophilic regions to which the probe biopolymers are immobilized by carrying out hybridization with the microarray shown in Fig. 1, therefore, these solutions do not flow out from the hydrophilic regions, whereby these solutions are not contaminated also when solutions containing different types of sample biopolymers are dripped on the respective hydrophilic regions. Consequently, solutions containing a plurality of types of (24 types at the maximum when employing the exemplary microarray shown in Fig. 1) different sample biopolymers can be selectively hybridized on a single microarray.

Fig. 3 schematically shows an exemplary closed vessel (hybridization chamber) for carrying out the hybridization method according to the present invention. The closed chamber basically comprises a lower housing 7 and an upper housing 8, for example, so that an internal space providing a sealed state is formed between the upper and lower housings 8, 7 when these housings 8, 7 are superposed with each other. The exemplary closed vessel shown in Fig. 3 is so constituted that an O-ring 9 of rubber is mounted on the lower housing 7 and the internal space formed by the upper and lower housings 8, 7 is sealed when these housings 8, 7 are superposed with each other.

The hybridization method employing this closed vessel is carried out in the following procedure, for example: First, the solutions 5 containing the sample biopolymers are dripped on the hydrophilic regions of the aforementioned microarray. The microarray 1 is placed on the lower housing 7. The lower housing 7 has a recess partially forming the internal space when superposed with the upper housing 8 as described above, and this recess stores a humectant 6 having the same vapor pressure as the aforementioned solutions 5 containing the sample biopolymers. The upper housing 8 is superposed on the lower housing 7, to seal the microarray 1 in the internal space. In this state, the sample biopolymers and the probe biopolymers are hybridized with each other by holding the closed vessel at a temperature of about 65°C for 8 to 16 hours. When the sample biopolymers are bound to the probe biopolymers, the sample biopolymers are immobilized onto the microarray along with the probe biopolymers. The hybridized sample biopolymers can be detected by taking out the microarray from the closed vessel after the hybridization, cleaning the microarray by dipping the microarray in a cleaning solution (exemplary composition of the cleaning solution: 0.2 × SSC solution and 0.5 × SSC solution containing 0.1 % of SDS) as such, thereafter exciting fluorescent materials labeled on the sample biopolymers immobilized onto the microarray with excitation light from a light source through a microarray scanner or the like, for example, and detecting emitted fluorescence.

In the present invention, the aforementioned closed vessel, illustratively comprising the upper housing 8 and the lower housing 7 prepared by aluminum die casting, for example, may be a vessel capable of sealing the microarray, and kitchen Tupperware for preserving food, for example, may be employed as the closed vessel.

The present invention also provides a hybridization microarray formed by arranging a plurality of hydrophilic regions to which a plurality of probe biopolymers are immobilized with a hydrophobic region to which no probe biopolymer is immobilized formed around the arranged plurality of hydrophilic regions. The aforementioned microarray is a novel once, and can be particularly preferably used for the aforementioned hybridization method according to the present invention.

The present invention further provides a hybridization kit comprising a microarray formed by arranging a plurality of hydrophilic regions to which a plurality of probe biopolymers are immobilized with a hydrophobic region to which no probe biopolymer is immobilized formed around the arranged plurality of hydrophilic regions and a closed vessel having an internal space capable of airtightly storing the said microarray. In other words, the combination of the microarray and the closed vessel preferably employable for the aforementioned hybridization method according to the present invention can be provided in the form of a kit. The solutions containing the sample biopolymers and the humectant can be preferably combined with each other as described above and particularly preferably employed for the aforementioned hybridization method according to the present invention by employing this kit. The kit according to the present invention may further have a humectant previously storable in the vessel, and may also be implemented to be capable of properly preparing a solution containing a sample biopolymer by dissolving the sample biopolymer in this humectant, as a matter of course.

While the present invention is now more specifically described, the present invention is not restricted to these Examples.

### <Example 1 >

Total RNA of 10 µg was prepared from 1 × 10⁶ HeLa cells with RNeasy Mini Kit (by Qiagen). Cy3-labeled HeLa cDNA was prepared from the prepared HeLa Total RNA of 10 µg with Labelstar Array Kit (by Qiagen) and Cy3-dUTP (by Amersham Biosciences) as sample biopolymers. The sample biopolymers were adjusted to a quantity of 120 µL with a 5 × SSC solution containing 0.5 % of SDS (solute molar concentration of the solution containing the sample biopolymers: 1.67 mol/L).

The solution containing the sample biopolymers prepared in the aforementioned manner was dripped onto hydrophilic regions of a microarray having 24 hydrophilic regions and a hydrophobic region formed to enclose these hydrophilic regions on the surface thereof with four probe biopolymers immobilized to each hydrophilic region. 70mer probes (synthesized in a DNA synthesizer) of Glyceraldehyde-3-phosphate dehydrogenase, Endonuclease G, pU 18 and Lambda phage DNA having base sequences shown in sequence numbers 1 to 4 of a sequence listing respectively were employed as the probe biopolymers immobilized to the hydrophilic regions.

After dripping the solution containing the sample biopolymers, the microarray was placed on the lower housing 7 of the closed vessel (hybridization chamber) comprising the lower housing 7 and the upper housing 8 shown in Fig. 3. The recess of the lower housing 7 previously stored 600 µL of 5 × SSC containing 0.5% of SDS as a humectant (solute molar concentration of humectant: 1.67 mol/L, difference in solute molar concentration from solution containing sample biopolymers: ±0 %). The upper housing 8 was superposed on the lower housing 7 for sealing up the microarray 1 in the internal space and the closed vessel was held at a temperature of 65°C for 14 hours, for hybridizing the sample biopolymers and the probe biopolymers with each other.

When the microarray was taken out from the closed vessel after the hybridization and the quantity of the solution containing the sample biopolymers was measured, the quantity was reduced by 5.5 µL than that at the point of dripping, and liquid change was 4.6 %. Thereafter the microarray was dipped in a cleaning solution (0.2 × SSC solution and 0.5 × SSC solution containing 0.1 % of SDS) to be cleaned, and Cy3 labeled on the sample biopolymers immobilized onto the microarray was thereafter excited with excitation light from a light source through a microarray scanner for detecting emitted fluorescence, whereby the hybridized biopolymers could be detected. Thus, it was possible to complete the hybridization in the state that the solution containing the sample biopolymers was in contact with only the slide glass to which the probe biopolymers were immobilized without drying the solution containing the sample biopolymers on the microarray.

### <Example 2>

Hybridization was carried out similarly to Example 1, except that 600 µL of 4.5 × SSC containing 0.5 % of SDS (solute molar concentration of humectant: 1.50 mol/L, difference in solute molar concentration from solution containing sample biopolymers: -10 %) was employed as a humectant. When the quantity of the solution containing the sample biopolymers was measured after the hybridization, the quantity was increased by 4.9 µL as compared with that at a point of dripping, and liquid change was 4.1 %. Thus, it was possible to complete the hybridization in a state that the solution containing the sample biopolymers was in contact with only a slide glass to which probe biopolymers were immobilized without increasing the volume of the solution containing the sample biopolymers and overflowing this solution from hydrophilic regions.

### <Example 3 >

Hybridization was carried out similarly to Example 1, except that 600 µL of 5.4 × SSC containing 0.5 % of SDS (solute molar concentration of humectant: 1.80 mol/L, difference in solute molar concentration from solution containing sample biopolymers: +8 %) was employed as a humectant. When the quantity of the solution containing the sample biopolymers was measured after the hybridization, the quantity was reduced by 12.0 µL as compared with that at a point of dripping, and liquid change was 10.0 %. Thus, it was possible to complete the hybridization in a state that the solution containing the sample biopolymers was in contact with only a slide glass to which probe biopolymers were immobilized without drying the solution containing the sample biopolymers on a microarray.

### <Comparative Example 1>

Hybridization was carried out similarly to Example 1, except that 600 µL of 4 × SSC containing 0.5 % of SDS (solute molar concentration of humectant: 1.33 mol/L, difference in solute molar concentration from solution containing sample biopolymers: -20 %) was employed as a humectant. When the quantity of the solution containing the sample biopolymers was measured after the hybridization, the quantity was increased by 17.4 µL as compared with that at a point of dripping (liquid change: 14.5 %), and the quantity of the solution containing the sample biopolymers was so remarkably increased that this solution overflowed and the hybridization could not be normally carried out.

### <Comparative Example 2>

Hybridization was carried out similarly to Example 1, except that 600 µL of 5.5 × SSC containing 0.5 % of SDS (solute molar concentration of humectant: 1.83 mol/L, difference in solute molar concentration from solution containing sample biopolymers: +10 %) was employed as a humectant. When the quantity of the solution containing the sample biopolymers was measured after the hybridization, the quantity was reduced by 14.3 µL as compared with that at a point of dripping (liquid change: 11.9 %), and the quantity of the solution containing the sample biopolymers was so remarkably reduced by vapor diffusion during the hybridization that the hybridization could not be normally performed.

### <Comparative Example 3>

Hybridization was carried out similarly to Example 1, except that 600 µL of 6 × SSC containing 0.5 % of SDS (solute molar concentration of humectant: 2.00 mol/L, difference in solute molar concentration from solution containing sample biopolymers: +20 %) was employed as a humectant. When the quantity of the solution containing sample biopolymers was measured after the hybridization, the quantity was reduced by 21.9 µL as compared with that at a point of dripping (liquid change: 18.2 %), and the quantity of the solution containing the sample biopolymers was so remarkably reduced by vapor diffusion during the hybridization that the hybridization could not be normally carried out.

Table 1 shows the aforementioned results of Examples 1 to 3 and comparative examples 1 to 3.

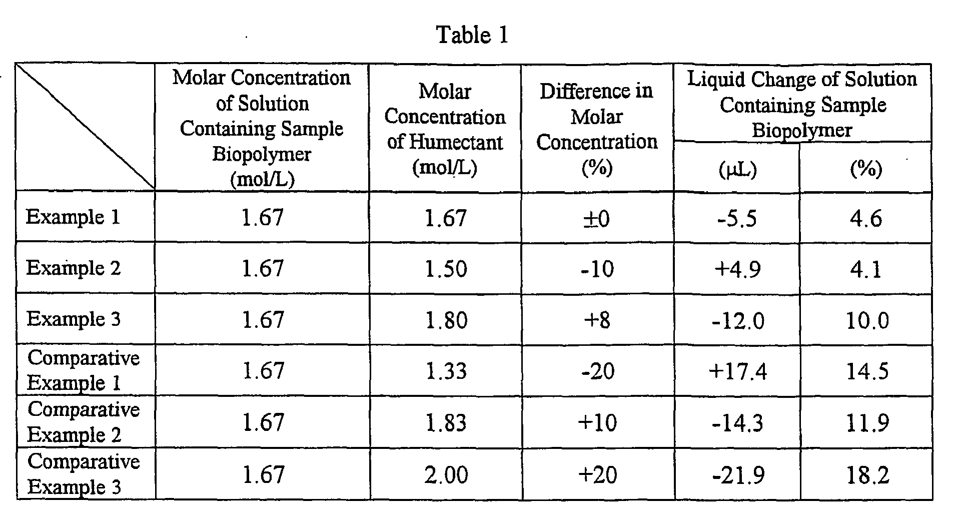

### Industrial Applicability

According to the inventive hybridization method, no structure on a slide glass such as a glass coverslip generally required for effectively carrying out hybridization is used and hence hybridization can be extremely simply and reliably carried out. Further, simultaneous multi-specimen hybridization of simultaneously detecting a plurality of types of sample biopolymers can be simply and reliably carried out by employing the hybridization microarray and the hybridization kit according to the present invention. Thus, the treatment time for a large number of samples as well the number of necessary microarrays and the quantity of required reagents can be remarkably reduced as compared with the prior art.

## Claims

1. A hybridization method of hybridizing a sample biopolymer and a probe biopolymer in a state that a solution containing the sample biopolymer is in contact with only a slide glass to which the probe biopolymer is immobilized, by carrying out hybridization in a closed vessel containing a solution having the same vapor pressure as the solution containing the sample biopolymer.

2. The hybridization method according to claim 1, carrying out hybridization on a slide glass constituted of a hydrophilic region having a surface to which a plurality of probe biopolymers are immobilized and a hydrophobic region, to which no probe biopolymer is immobilized, around the hydrophilic region.

3. The hybridization method according to claim 2, wherein the slide glass is a microarray formed by arranging a plurality of hydrophilic regions to which a plurality of probe biopolymers are immobilized with a hydrophobic region to which no probe biopolymer is immobilized formed around the arranged plurality of hydrophilic regions.

## Patentansprüche

1. Hybridisierungsverfahren zur Hybridisierung eines Proben-Biopolymers und eines Sonden-Biopolymers derart, dass sich eine das Proben-Biopolymer enthaltende Lösung nur mit einem Objektträger, auf dem das Sonden-Biopolymer immobilisiert ist, in Kontakt befindet, wobei die Hybridisierung in einem geschlossenen Gefäß durchgeführt wird, das eine Lösung mit demselben Dampfdruck wie die das Proben-Biopolymer enthaltende Lösung enthält.

2. Hybridisierungsverfahren nach Anspruch 1, wobei die Hybridisierung auf einem Objektträger durchgeführt wird, der einen hydrophilen Bereich, an den eine Vielzahl von Sonden-Biopolymeren immobilisiert sind, und um den hydrophilen Bereich einen hydrophoben Bereich, an den keine Sonden-Biopolymere immobilisiert sind, aufweist.

3. Hybridisierungsverfahren nach Anspruch 2, wobei der Objektträger einen Mikroarray darstellt, der gebildet wird, indem eine Vielzahl von hydrophilen Bereichen, an die eine Vielzahl von Sonden-Biopolymeren immobilisiert sind, angeordnet werden, und ein hydrophober Bereich, an den keine Sonden-Biopolymere immobilisiert sind, um die Vielzahl der hydrophilen Bereiche gebildet wird.

## Revendications

1. Procédé d'hybridation pour hybrider un biopolymère d'échantillon et un biopolymère de sonde à un état tel qu'une solution contenant le biopolymère d'échantillon soit en contact avec seulement une lame de verre au niveau de laquelle le biopolymère de sonde est immobilisé, en effectuant l'hybridation dans un récipient clos hermétiquement contenant une solution ayant la même pression de vapeur que la solution contenant le biopolymère d'échantillon.

2. Procédé d'hybridation suivant la revendication 1, mettant en oeuvre l'hybridation sur une lame de verre constituée d'une région hydrophile ayant une surface à laquelle est immobilisée une pluralité de biopolymères de sonde, et une région hydrophobe, à laquelle aucun biopolymère de sonde n'est immobilisé, autour de la région hydrophile.

3. Procédé d'hybridation suivant la revendication 2, dans lequel la lame de verre est un microréseau formé en installant une pluralité de régions hydrophiles à laquelle est immobilisée une pluralité de biopolymères de sonde avec une région hydrophobe à laquelle aucun biopolymère de sonde n'est immobilisé, formée autour de la pluralité installée de régions hydrophiles.
